# EUROPEAN PATENT SPECIFICATION

(11) **EP 4 348 270 B1**
(45) Date of publication and mention of the grant of the patent: **18.11.2026**
(21) Application number: 22811734.7
(22) Date of filing: 18.05.2022
(51) Int. Cl.: G01N 33/98, G01N 21/3504, G01N 33/497, A61B 5/00, A61B 5/08

(54) **METHOD AND SYSTEM FOR PREVENTING TAMPERING OF BREATH SAMPLE MEASUREMENTS**
VERFAHREN UND SYSTEM ZUR VERHINDERUNG DER MANIPULATION VON ATEMPROBENMESSUNGEN
PROCÉDÉ ET SYSTÈME POUR EMPÊCHER L'ALTÉRATION DE MESURES D'ÉCHANTILLON D'HALEINE

(30) Priority: 28.05.2021 SE 2150681
(43) Date of publication of application: 10.04.2024
(73) Proprietor: Automotive Coalition for Traffic Safety, Inc., Ashburn, VA 20147 (US)
(72) Inventor: HÖK, Bertil, 723 44 VÄSTERÅS (SE); LJUNGBLAD, Jonas, 112 56 Stockholm (SE)
(74) Representative: Zacco Sweden AB
(86) International application number: PCT/SE2022/050488
(87) International publication number: WO 2022/250595

(56) References cited:
- EP-A1- 2 888 588
- EP-A1- 2 969 633
- EP-A1- 3 106 872
- EP-A1- 3 106 872
- EP-B1- 2 100 763
- EP-B1- 2 969 633
- US-A1- 2013 141 574
- US-A1- 2018 074 081
- US-B2- 10 506 949

## Description

### Field of the invention

The present invention relates to a breath analyzing device and method. In particular the invention relates to a breath analyzing device and method reducing the possibilities to tamper with the results without increasing the analyzing time or inconvenience for the user.

### Background of the invention

Breath analyzing equipment is becoming increasingly common, not the least in vehicles as a measure to detect and prevent driving under the influence of intoxicating substances, in particular, ethyl alcohol (ethanol). The breath analyzing equipment may be a stand-alone, even handheld, unit that gives a measured value of the content of a substance or substances in the driver's breath. Alternatively, breath analyzing equipment may be part of a system wherein also including equipment for identifying the driver and/or immobilizing the vehicle. Such breath analyzing equipment is typically permanently mounted in the vehicle and may be an integral part of the dashboard, for example. Breath analyzing equipment may also be stationary systems used to control access to a work area, a vehicle fleet depot or the like. To provide a breath analyzer that has appropriate sensitivity, is reliable and provides a reasonably fast analysis is far from trivial. This is especially true if the breath analyzing equipment should be able to detect a plurality of substances and not being disturbed by variation in moisture, CO₂ content etc. Breath analyzing equipment that fulfills these requirements are described in for example US7919754 and US9746454.

The breath analyzing equipment may be part of a system also including equipment for identifying the driver and/or immobilizing the vehicle, so called "alcolocks". Such breath analyzing equipment is typically permanently mounted in the vehicle and may be an integral part of the dashboard, for example, and connected to the control system of the vehicle. Alcolocks are in widespread use in offender programs as a mandatory accessory for rehabilitation of car owners who have been convicted for drunk driving. In addition, similar systems and devices are being used in commercial vehicles like buses, taxis and trains.

However, it appears that these systems will also be common in private vehicles in a near future, and possibly also mandatory in at least some countries and regions.

The up to the present day most common approach for both vehicle mounted breath testing equipment and stationary testing stations is to use a mouthpiece to which, after a deep breath, the user should empty his or her airways. This approach is referred to as active detection. To ensure a correct determination the user should deliver a forced expiration at almost full vital capacity. This requires substantial time and effort, especially for persons with limited capacity. In addition, the mouthpiece, or part of the mouthpiece, is often a disposable plastic item for hygienic reasons. This results in cumbersome handling and the use of vast amounts of disposable plastic items which is questionable from an environmental viewpoint.

Contactless detection is an alternative approach is wherein no mouthpiece is utilized and the breath testing apparatus typically receives a mixture of the exhaled breath and the surrounding air and a detection of an intoxicating substance is determined from a breath sample taken during the expiration at normal breathing. The detection may be truly passive wherein no action is required of the user, for example taking place while a user performs the regular starting up routine of a vehicle. Alternatively, the user may be instructed to perform certain actions that are meant to facilitate the detection process, for example the user may be instructed to breath towards an air inlet or the like. One challenge with contactless detection, even if user is instructed to breath in a certain direction or the like, is the low concentration of the substances to be detected and analyzed. An established method is to utilize tracer gases, typically carbon dioxide or water vapor, which are always present in the breath in highly predictable amounts, to both trigger the analysis of the target substance and to facilitate the determination of the target substance concentration value. However, it has proven difficult to get the contactless detection to function in a satisfactory manner in real life scenarios. To correctly analyze the concentration of an intoxicating substance may take considerable time. The time required may be perceived as unacceptable long for example for a drive starting up a vehicle or for an employer about to enter a gated workplace.

One further challenge with contactless breath analyzer systems is that such systems may be particularly susceptible to tampering. Tampering may for example include cooling down the breath sample with a cooled tube or filtering the breath sample in charcoal. Tampering is a recognized problem and has been addressed in various ways. However, the known approaches typically add considerably complexity to the breath analyzer system, makes the use more cumbersome or increase the time needed to deliver a result to the user.

EP 3106872 A1 discloses a system and method wherein the breath analyzer system is provided with a camera and the produced images are analyzed with advanced image analysis methods to detect and warn for tampering attempts.

US 2018/074081 A1 discloses an authentication system and authentication method for detecting breath alcohol.

EP 2100763 B1 discloses a method and device for monitoring a motorised device and mobile device.

EP 2969633 B1 discloses anti-circumvention apparatus and methods for use in sobriety testing systems.

### Summary of the invention

The invention is defined by the independent claims. Furthermore, the embodiments of the invention are those defined by the claims. Moreover, examples and embodiments, which are not covered by the claims, are presented not as embodiments of the invention, but as background art or examples useful for understanding the invention.

The object of the invention is to provide a breath analyzing system and method of operation that overcomes the drawbacks of prior art contactless detection systems.

This is achieved by the method as defined in the independent method claim, and the breath analysis device as defined in the independent device claim.

According to one aspect of the invention a method is provided. The method for determining the validity of a measurement of a concentration of an intoxicating substance in the exhaled breath of a user, utilizes a breath analysis device comprising:
a measuring cell arranged to sample a sensor signal representing the concentration of the intoxicating substance and a sensor signal representing the concentration of a tracer substance; and
a line-of-sight detector with a predetermined field of view and arranged to measure the coverage by an object of the field of view and to output a signal representing the degree of coverage.

The method comprises the steps of:
- monitoring the output signal of the line-of-sight detector, and if the output signal deviates from an established background value record the line-of-sight detector output signal as a function of time, the output signal variation being a recorded line-of-sight signal signature;
- monitoring the tracer substance sensor signal and if a peak in the tracer substance is detected, the peak indicating a possible exhalation phase of the respiratory cycle of the user determine a breath concentration value of the intoxicating substance based on the intoxicating substance sensor signal and the tracer substance sensor signal;
- comparing the recorded line-of-sight signal signature originating from the line-of-sight detector with at least one stored reference signal signature;
- comparing the relation between the line-of-sight detector output signal and the tracer substance signal with stored signal relation criteria; and wherein
- the validity of the measurement of the breath concentration value is confirmed if the recorded line of sight signal signature matches the stored reference signal signature and if the recorded line-of-sight output signal and the tracer substance signal fulfils the stored signal relation criteria.

According to one embodiment of the invention in the monitoring step a background value of the line-of-sight detector output signal is determined, and the step of comparing the recorded line of sight signal signature with the stored reference signal signature comprises at least comparing one or a selection of the parameters: signal slope after an initial ascension, duration of a time period of increasing signal, a signal value associated to the upper value, duration of the signal at or above a predefined level relating to the upper level, and signal slope after a peak or plateau.

According to one embodiment of the invention the stored signal relation criteria comprise a time relation between the line-of-sight detector output signal and the tracer substance signal. The time relation criterion may require that an increase in the recorded line-of-sight detector output signal, indicative of an object approaching the breath analysis device occurs before the onset of the peak in the tracer substance signal. The time relation criterion may further require that a peak in the recorded line-of-sight detector output signal coincides with the peak in the tracer substance signal within a predetermined time period. Further, the step of comparing the relation between the line-of-sight detector output signal and the tracer substance signal with stored signal relation criteria may comprise correcting the time difference of the peak in the line-of-sight detector output signal and the peak in the tracer substance by a predetermined factor relating to an expected time delay relating to the time required for an exhaled breath sample reaching the breath analysis device. Alternatively, the time relation between the line-of-sight detector output signal and the tracer substance signal may include an expected time differences between the peak in the line-of-sight detector output signal and the peak in the tracer substance by a predetermined factor relating to an expected time delay relating to the time required for an exhaled breath sample reaching the breath analysis device.

According to one embodiment of the invention the line-of-sight detector is arranged to measure heat radiation. The breath analysis device may further comprise means for measuring ambient temperature, and wherein the output signal from the line-of-sight detector is compensated with the ambient temperature.

According to one aspect of the invention a breath analysis device is provided. The breath analysis device comprises:
- a measuring cell arranged to sample a sensor signal representing the concentration of the intoxicating substance and a sensor signal representing the concentration of a tracer substance;
- a line-of-sight detector with a predetermined field of view and arranged to measure the coverage by an object of the field of view and to output a signal representing the degree of coverage;
- a control and signal processing unit in connection with the measuring cell and the line-of-sight detector, wherein the breath analysis device is arranged to perform the steps of:
   - monitoring the output signal of the line-of-sight detector, and if the output signal deviates from an established background value record the line-of-sight detector output signal as a function of time, the output signal variation being a recorded line-of-sight signal signature;
   - monitoring the tracer substance sensor signal and if a peak in the tracer substance is detected, the peak indicating a possible exhalation phase of the respiratory cycle of the user determine a breath concentration value of the intoxicating substance based on the intoxicating substance sensor signal and the tracer substance sensor signal;
   - comparing the recorded line-of-sight signal signature originating from the line-of-sight detector with at least one stored reference signal signature;
   - comparing the relation between the line-of-sight detector output signal and the tracer substance signal with stored signal relation criteria; and wherein
   - the validity of the measurement of the breath concentration value is confirmed if the recorded line of sight signal signature matches the stored reference signal signature and if the recorded line-of-sight output signal and the tracer substance signal fulfils the stored signal relation criteria.

According to one embodiment of the invention the line-of-sight detector is arranged to detect electromagnetic radiation and comprises an aperture determining an effective field of view of the line-of-sight detector. The line-of-sight detector may comprise a sensor utilizing infra-red detection and is arranged to measure heat radiation.

According to one embodiment of the invention the sensor of the line-of-sight detector (108) is an active sensor arranged to utilize near infrared reflectance measurements.

According to one embodiment of the invention the line-of-sight detector is arranged to have a field of view corresponding to a predefined area at a predefined distance from the breath analysis apparatus, wherein the predefined area is the area of a typical human face of a person using the breath analysis device at the predefined distance, the predefined distance associated with appropriate use of breath analysis device and being between 100-300 mm.

According to one embodiment of the invention the breath analysis device further comprises means for measuring ambient temperature, and wherein a signal from the line-of-sight detector is compensated with the ambient temperature.

According to one embodiment of the invention the line-of-sight detector comprises a plurality of sensor arranged to provide a corresponding plurality of output signals providing a spatial resolution of the field of view which may form a low resolution image of the object in the field of view. The line-of-sight detector may comprise a 8x8 matrix of IR photodetectors.

According to one embodiment of the invention the breath analysis device is further arranged to, in the monitoring step, determine a background value of the line-of-sight detector output signal is, and in the step of comparing the recorded line of sight signal signature with the stored reference signal signature comparing one or a selection of the parameters: signal slope after an initial ascension, duration of a time period of increasing signal, a signal value associated to the upper value, duration of the signal at or above a predefined level relating to the upper level, and signal slope after a peak or plateau.

According to one embodiment of the invention the breath analysis device is further arranged to store signal relation criteria which comprises a time relation between the line-of-sight detector output signal and the tracer substance signal. The time relation criterion may require that an increase in the recorded line-of-sight detector output signal, indicative of an object approaching the breath analysis device occurs before the onset of the peak in the tracer substance signal. The time relation criterion may further require that a peak in the recorded line-of-sight detector output signal coincide with the peak in the tracer substance signal within a predetermined time period. Further, the step of comparing the relation between the line-of-sight detector output signal and the tracer substance signal with stored signal relation criteria may comprise correcting the time difference of the peak in the line-of-sight detector output signal and the peak in the tracer substance by a predetermined factor relating to an expected time delay relating to the time required for an exhaled breath sample reaching the breath analysis device. Alternatively, the time relation between the line-of-sight detector output signal and the tracer substance signal may include an expected time difference-between the peak in the line-of-sight detector output signal and the peak in the tracer substance by a predetermined factor relating to an expected time delay relating to the time required for an exhaled breath sample reaching the breath analysis device.

Thanks to the invention it is possible to provide a breath analysis method and breath analysis device that can validate a measurement of a concentration of an intoxicating substance in the exhaled breath of a user.

One advantage afforded by the present invention is that the validation can be performed with a stand-alone unit not relying on external databases nor complicated image analysis systems.

A further advantage is that the validation is fast and reliable, which is of great importance in getting acceptance for implementing breath analysis systems and routines in for example workplaces.

Yet a further advantage with the breath analysis method and breath analysis device according to the invention is that the method and device can be implemented in various ways, including, but not limited to, handheld devices, stationary devices provided for example in an entrance to a work area and vehicle mounted devices. The breath analysis device may readily be incorporated in the dashboard of a vehicle and utilize existing functions and facilities provided in the cars infotainment system, for example. The breath analysis device may also be in communicating connection with a vehicle immobilizing system, a so called alco-lock.

In the following, the invention will be described in more detail, by way of example only, with regard to non-limiting embodiments thereof, reference being made to the accompanying drawings.

### Brief description of the drawings

Figures 1a-b are schematic illustrations of a breath analysis device according to the present invention wherein a) illustrates the breath analysis device; and b) illustrates the basic concepts of line-of-sight and field of view;
Figures 2a-c are schematic graphs illustrating sensors signals of the breath analysis device according to the present invention, wherein a) shows graphs of typical sensor signals of the breath analysis device including the line-of-sight detector (upper graph) and tracer and substance signal (lower graph), b) are graphs and schematic illustrations showing the relation between signal variations and field of view of line-of-sight detector, and c) illustrates a typical signal signature of the line-of-sight detector and its quantification;
Figure 3 is a flowchart of the method according to the invention;
Figures 4a-c are schematic drawings of different embodiments of the line-of-sight detector arrangement according to the invention;
Figure 5 is a graph of the spectral properties of one embodiment of line-of-sight detector based on infrared thermometry;
Figure 6 illustrates one embodiment of a line-of-sight detector based on reflective near infrared radiation (NIR) including a graph of its field of view;
Figure 7 shows the field of view provided by one embodiment of a line-of-sight detector employing a light emitting diode and photodiode monitoring the optical reflectance within a predefined field of view; and
Figures 8a-c illustrates schematically different application examples of the breath analysis device according to the invention, including a) handheld device, b) wall mounted device, and c) automotive vehicle integrated unit.

### Detailed description

Terms such as "top", "bottom", upper", lower", "below", "above" etc are used merely with reference to the geometry of the embodiment of the invention shown in the drawings and/or during normal operation of the device/devices and are not intended to limit the invention in any manner.

### Definitions:

A tracer is a physiological substance inherently associated with expired breath, e g carbon dioxide or water vapor.

By baseline is meant a signal level corresponding to the concentration of intoxicating substance or tracer to which other instantaneous signal values are referred. An offset error is a deviation from the baseline.

A concentration peak is defined by a maximum in a measured concentration versus time with increasing concentration before the peak maximum and declining thereafter.

The breath analysis device and the method according to the invention will primarily be described as a contactless detection system mounted in a vehicle, which represents an important implementation of the invention. The breath analysis device may be fully integrated in the vehicle, for example utilizing an existing vehicle infotainment system for communication with a user and/or the HVAC system for directing air flows into the breath analysis device, for example. Integrated systems breath analysis devices are described in SE1950840-7. A vehicle breath analysis device according to the invention may also be retrofitted in the vehicle, in which case the breath analysis device would typically be more independent from other parts of the vehicle instrumentation. As realized by the skilled person, the teachings are equally relevant for stand alone systems, for example systems at an entrance to a work area, fleet depot or the like. The breath analysis device and the method according to the invention may also be a handheld system for use in a vehicle or elsewhere.

The breath analysis device and method according to the invention may readily be combined with recently developed systems and methods for improving the speed and performance of breath analysis device, for example as described in SE1950840-7 and SE2050105-2.

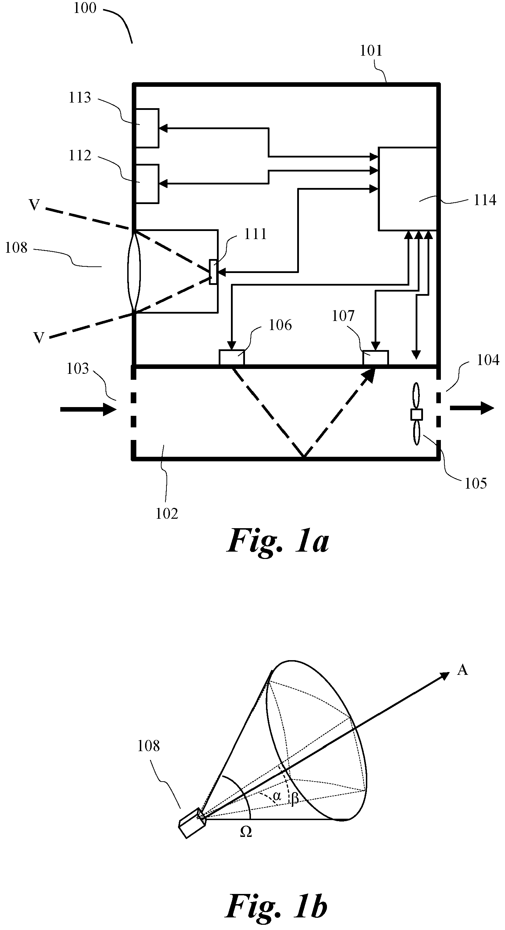

Figure 1a is a schematic illustration of the breath analysis device 100 according to the invention. The breath analysis device 100 comprises a housing 101, a measuring cell 102, a line-of-sight detector 108, one or more audiovisual communicating units 113 and a control and signal processing unit 114. The breath analysis device 100 is adapted to analyze the exhaled breath from a user within a predefined maximum distance. By determining the concentration of both an intoxicating substance and a tracer gas at this distance, the breath concentration of an intoxicating substance can be determined. The tracer gas has a known breath concentration, and by measuring its concentration at the location of the measuring cell 102, the dilution of the exhaled breath can be determined. Typically, carbon dioxide (CO₂) or water vapor (H₂O) are being used as tracer gas, exhibiting nominal concentrations of 4.2 and 5.6 percent by volume in the exhaled breath of human beings, respectively.

The measuring cell 102 is provided with an inlet 103 and an outlet 104 in the housing 101 and means to force air from the inlet 103 to the outlet 104, for example a fan 105. The measuring cell 102 is thus during operation continuously exposed to air withdrawn from the position of the breath sampling inlet 103. The inlet 103 is arranged is arranged on a surface of the housing 101 that during operation of the breath analysis device 100 faces the user.

In implementation wherein the breath analysis device 100 is provided in a vehicle the breath analysis device 100 may be fully integrated in for example a dashboard, in which case the housing 101 would not be a separate part. Also with a fully integrated device, the breath analysis device 100 may not have dedicated audiovisual communicating units 113 and a dedicated control and signal processing unit 114, rather utilizing such capacities provided in the vehicles. These units should hence be regarded as functional units in integrated implementations.

The measuring cell 102 is preferably operating by the principle of infrared (IR) spectroscopy. An IR emitter 106 transmits a beam of IR radiation which is being absorbed by an IR detector 107 after reflections against the interior walls of the measuring cell 102. The surfaces of the interior walls are preferably coated with a highly reflective material, for example aluminum. The number of reflections and the collimation of the IR beam can be adapted to the expected range of IR absorption coefficients and concentrations of tracer and intoxicating substances. The emitter 106 is typically a blackbody membrane having small mass, and therefore being adapted to emit pulsating radiation in a broad wavelength range of 3-10 µm with a repetition frequency of 5 Hz or more. The IR detector 107 includes narrowband filters tuned to absorption peaks of one or several intoxicating substances, for example ethyl alcohol, having an absorption peak at a wavelength of 9.5 µm, and to one or several tracer gases, such as carbon dioxide and water vapor exhibiting absorption peaks at 4.3, and 6.0 µm, respectively. The presence of the intoxicating substance or the tracer gas will result in a decreasing IR intensity transformed into an electrical signal by the multichannel IR detector 107, having optical filters adapted to the specific absorption peaks, and operating synchronously with the repetition frequency of the IR emitter 106. As appreciated by the skilled person also other types of measuring cells, operating according to different principles, may be utilized. The choice of measuring cell or measuring principle may for example depend on the specific intoxicating substance or substances to be analyzed or construction constrains such as power consumption or cost. The present invention is not limited to a specific measuring cell or measuring principle with regards to the measurement of the breath concentration of a specific breath concentration of an intoxicating substance.

The line-of-sight detector 108 is arranged to provide information if an object is present and correctly positioned with regards to the inlet 103 in order for a breath sample to be correctly analyzed. This will be referred to as the line-of-sight detector 108 is arranged to provide an object presence signal. The line-of-sight detector 108 is further arranged to provide information regarding the nature of the object, i.e. if the object is a living human being or not, which will referred to as a signal signature. A plurality of different measuring techniques implemented by various line-of-sight detectors will be further discussed below.

As schematically illustrated in Figure 1b the line-of-sight detector 108 has defined effective field of view indicated with *Ω*. The field of view is typically provided as a solid angle quantified in steradian. Alternatively, the field of view may be given as an angle α in a horizontal or vertical direction or a combination of angles α and β, in the horizontal and vertical directions, respectively. A line-of-sight detector typically has an inherent field of view from its basic design. The inherent field of view may be altered by how the line-of-sight detector 108 is mounted in the housing 101 and by providing means such as one or more lenses and/or an aperture in front of the line-of-sight detector 108 giving the effective field of view *Ω*. The skilled person, having a line-of-sight detector with an inherent field of view would know how to provide means to alter the angle of view to a desired effective-field of view. The field of view is typically fixed, but means for adjusting the field of view could be envisaged. The line-of-sight detector 108 as mounted in the housing 101 further has a direction of view , which defines a thought center line in the field of view, in Figure 1b indicated by arrow A. The direction of view is typically mainly dependent on how the line of sight detector 108 is mounted in the housing 101 and/or how the complete breath analysis device 100 is is mounted on a dashboard, for example. The line-of-sight detector 108 and the means for controlling the-field of view, if such are required, are typically arranged in the housing such that the effective field of view and the direction of view will match the position and coverage of the face of a user during normal operation of the breath analysis device 100. Typically a user is instructed to exhale towards the inlet 103 within a predefined maximum distance, typically 100-300 mm, between the subject's face and the inlet 103. A typical face with a height/width of 200mm respectively 150 mm would give the angles α and β in the range 20-100°. As discussed, the field of view and also the direction of view may be adjustable, for example by adjusting the geometry of the detector or the optical configuration. In one embodiment the field of view and/or the direction of view are parameters that are adjustable via the control and signal processing unit 114.

The line-of-sight detector 108 is typically positioned alongside the breath sampling inlet 3 of the measuring cell 102 on the outside surface 101a of the breath analysis device 101. The field of view *α* of the line-of-sight detector 108 is adapted to be encompassed by the subject's face at the predefined maximum distance. The line-of-sight detector 108 comprises a sensor 111, for example and typically a multilayer microstructure 111, providing an electrical signal corresponding to the average temperature, reflectance or other specific property of object within its field of view. The active area of the microstructure 111 is typically 0.3 x 0.3 mm. Its field of view is adapted to the predefined distance by an optical arrangement 110, typically a refractive lens.

According to one embodiment the breath analysis device 100 is provided with an auxiliary sensor 112 adapted to monitor the immediate environment, for example the ambient temperature. This may be advantageous for example in the case that the line-of-sight detector 108 is an IR-sensor and p may have some cross sensitivity to ambient temperature. By measuring both the effects of radiated and conducted heat and combining the signals, influence from the ambient temperature may be minimized. The auxiliary sensor 112 may be arranged to measure other ambient variables as well, including humidity, barometric pressure, and illuminance.

The breath analysis device 100 may further included or be connected to a power supply (not shown) and data communication means (not shown) for two-way transfer of information between the breath analysis device 101 and other electronic equipment, for example, means for controlling the drivability of a vehicle.

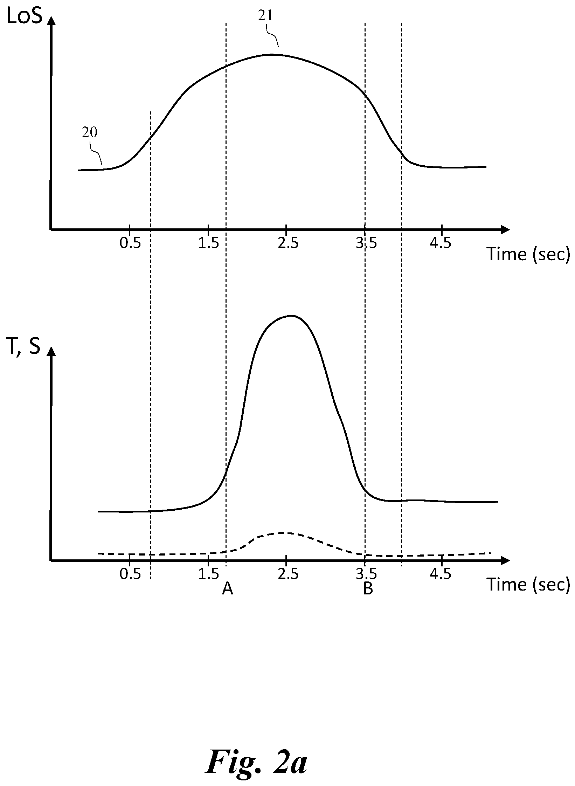

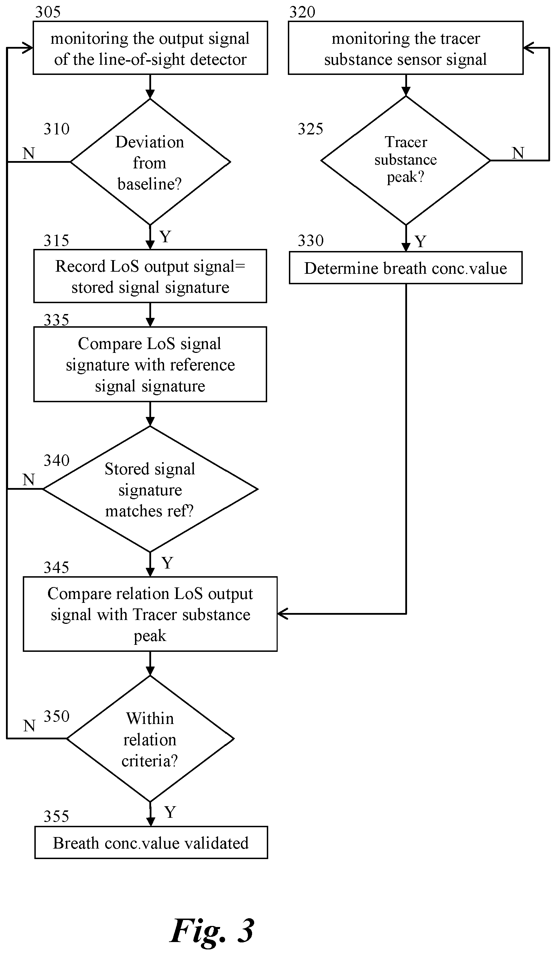

The functionality of the breath analysis device 100 and the method of validating a measurement of a concentration of an intoxicating substance in the exhaled breath of a user according to the invention will first be described in a general case, with reference to the schematic graphs of Figures 2a-c and to the flowchart of Figure 3, followed by different embodiments utilizing different detector technologies relating to the line-of-sight detector 108. Figure 2a represents the output signal from the line-of-sight detector 108 and the output from the measuring cell 102 wherein the upper curve, indicated with *T* relates to the tracer signal and the lower curve S, relates to the substance signal.

The breath analysis device 100 described with reference to figure 1a-b is arranged to perform the steps of the method. In particular, the measuring cell 102 of the breath analysis device 100 is arranged to sample a sensor signal representing the concentration of the intoxicating substance and a sensor signal representing the concentration of a tracer substance, preferably, but not exclusively, with the IR-based technology described above. The functionality controlled by, and the sensor/detectors signals processed by the control and signal processing unit 114. The line-of-sight detector 108 is arranged with a predetermined field of view *Ω* and arranged to measure the coverage by an object of the field of view and to output a signal representing the degree of coverage.

The method according to the invention, performed with the breath analysis device 100 and controlled by the control and signal processing unit 114, comprises the steps of:
305: -monitoring the output signal of the line-of-sight detector 108, and
   310: -if the output signal deviates from an established background value,
   315: -record the line-of-sight detector output signal as a function of time, the output signal variation being a recorded line-of-sight signal signature;
320: -monitoring the tracer substance sensor signal, and
   325: -if a peak in the tracer substance is detected, the peak indicating a possible exhalation phase of the respiratory cycle of the user,
   330: -determine a breath concentration value of the intoxicating substance based on the intoxicating substance sensor signal and the tracer substance sensor signal.

The validity of the measurement of the breath concentration value is determined by the steps of:
335-340: -comparing the recorded line-of-sight signal signature originating from the line-of-sight detector 108 with at least one stored reference signal signature to determine if the recorded line of sight signal signature matches stored reference signal signature and thereby indicating a human person approaching the breath analysis device 100;
345-350: -comparing the relation between the line-of-sight detector output signal and the tracer substance signal with stored signal relation criteria to determine if the recorded line-of-sight output signal has the expected relation with the tracer substance signal.
355: - The determined breath concentration value of the intoxicating substance is determined as valid measurement in the meaning of not having been tampered with if both the recorded line of sight signal signature matches stored reference signal signature and the line-of-sight detector output signal and the tracer substance signal fulfils the stored signal relation criteria.

The steps of monitoring 305 the output signal of the line-of-sight detector 108, and steps 310-315 to record the line-of-sight signal signature may typically include establishing a background or baseline value. This may be an output signal value sampled over one or a few seconds and averaged. A deviation from the background level, indicating an object being within the field of view of the line-of-sight detector 108 is detected and will initiate the recording of the line-of-sight signal signature. Ways to perform the detection will be further discussed below. The level 20 indicated in Figure 2a is a representation of a typical background signal and the upper level 21 is a representation of the signal resulting from an object being within the field of view. Depending on how long the object or person remains within the field of view of the line-of-sight detector 108 the upper level 21 may be in the form of a plateau, a broad peak or a narrow peak. The latter corresponding to a person leaning towards the breath analyzing device 100 and retracting from it in a more or less or continuous motion.

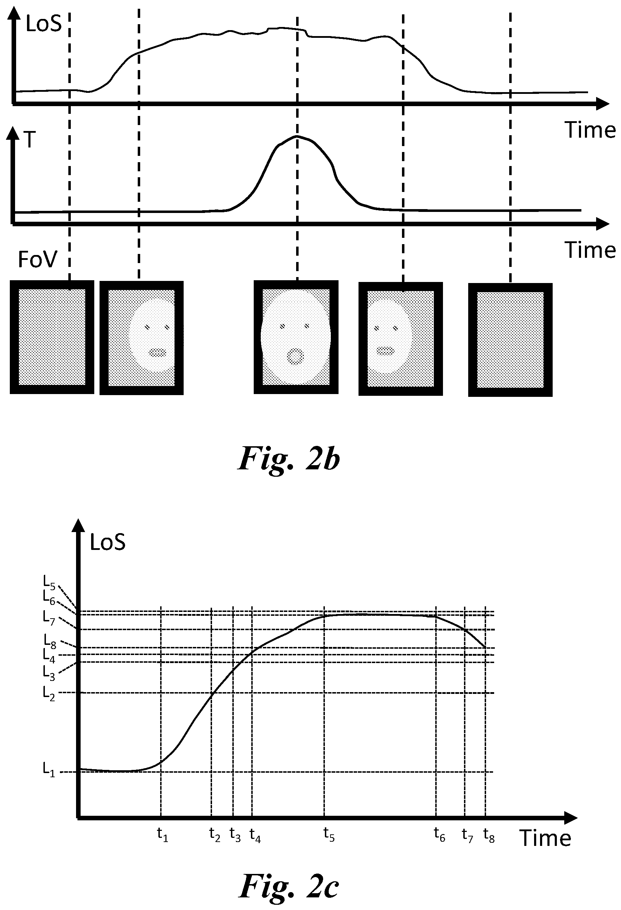

According to one embodiment the line-of-sight signal signature comprises a time variation in the signal on the upper level 21, illustrated in Figure 2b, which for example could be ascribed to the object moving or detecting an action by the object, for example an exhalation. For example, the heat variation caused by an exhalation could be detected by the line-of-sight detector 108 in the form of an IR-sensor.

According to embodiments of the invention in the steps of comparing 345-350 the relation between the line-of-sight detector 108 output signal and the tracer substance signal, the stored signal relation criteria comprise an expected time relation between the line-of-sight detector output signal and the tracer substance signal. This time relation criteria may require that an increase in the recorded line-of-sight detector output signal, indicative of an object approaching the breath analysis device 100 occurs before the onset of the peak 44 in the tracer substance signal. A time relation criterion may also be that the peak in the recorded line-of-sight detector output signal coincide with the peak in the tracer substance signal within a predetermined time period. This comparison may comprise correcting for the time difference of the peak in the line-of-sight detector output signal and the peak in the tracer substance by a predetermined factor relating to an expected time delay relating to the time required for an exhaled breath sample reaching the breath analysis device. Alternatively, this delay in time between the peak in the line-of-sight detector output signal and the peak in the tracer substance may in itself be a relation criterion, in that a predictable time delay may be associated with a correct and normal behavior of the user using the breath analysis device 100. On the other hand, a user trying to tamper the measurement, for example using CO₂ delivered from a tube close to the inlet 103, will result in a time delay different from the expected.

According to embodiments of the invention the steps of comparing 335-340 the recorded signature signal with the stored reference signal signature comprises at least comparing one or a selection of the parameters: signal slope after an initial ascension, duration of a time period of increasing signal, a signal value associated to the upper value 21 (plateau value), duration of the signal at or above a predefined level relating to the upper level 21, signal slope after a peak or plateau. Suitable parameters and how they may be utilized are schematically illustrated in Figures 2 b-c. Figure 2 b) provides an illustration of a line-of-sight LoS signal, a simultaneous tracer T signal and the occurrence of a human face appearing in the field of view of the line-of-sight detector. Initially, the field of view represents a background which may be unstructured as indicated or could be a structured but immobile surface. When a human face appears within the field of view, the line-of-sight signal is changing from a steady state background level and will reach an upper level typically in form of a plateau, or a broad peak, when most of the field of view is covered. Some signal variations may be observed at this upper level corresponding to small movements of the human face, and to variations in shape, for example mouth opening and exhalation. The tracer signal T typically exhibits a distinct peak coinciding with a human exhalation. Withdrawal of the human face from the field of view will bring the line-of-sight signal from the plateau level back to the background level.

Figure 2 c) depicts a signal line-of-sight from a line-of-sight detector as a function of time in some more detail. The signal can be divided into time segments which are each characterized by a signal slope characteristic of that segment. The transition from one segment to another is defined as the crossing of linear extensions between the segments, and the coordinates Lᵢ;tᵢ , i=1, 2, ... 8, in the example. The coordinates Lᵢ;tᵢ of this example defines the signal signature. Specifically, the almost flat time segment 0-t₁ represents a background signal, t₁-t₂ a sharp ascension coinciding with the object, normally the face of a person to be tested, entering the field of view, t₅-t₆ a plateau in which the object is filling the field of view, and t₆-t₇ the start of descension, when the object is moving from field of view. The additional time segments t₂-t₃, t₃-t₄, t₄-t₅, t₇-t₈ are intermediate states which may or may not be significant for the interpretation of the signal signature.

Identification of the Lᵢ;tᵢ coordinates is performed by a simple algorithm using differential calculus of consecutive time samples providing a value of the present slope, and comparing it with previous ones.

In the example described above and depicted in Figure 2 c) the stored reference signal signature will be the parameter set of the Lᵢ;tᵢ coordinates, i=1, 2, ... N, characteristic of (i) a background stationary phase (ii) and ascending phase (iii) a plateau (iv) and the start of descending phase. The set of reference Lᵢ;tᵢ coordinates will typically include tolerances for normal variability based on empirical data obtained from actual implementations of line-of-sight detectors.

Comparing one line-of-sight signal with another having a slightly different field of view, the two signals will obviously have almost equal appearance except for a small time delay due to the translational movement of the object entering or leaving the field of view. The coordinates Lᵢ;tᵢ will therefore slightly differ in time between these two signals in such a case. In contrast, a time variation of the shape of the object, for example mouth opening, will not provide a similar time difference of the Lᵢ;tᵢ coordinates. If several objects move independently of each other, the time differences between the Lᵢ;tᵢ will exhibit a less orderly time variation, indicating a case of manipulation attempt.

According to one embodiment the line-of-sight signal signature relates to the line-of-sight detector 108 additionally having a spatial resolution so that a change in the signal with time also can be ascribed to a specific area. For example, a simple array of IR-sensors could resolve a time dependent heat variation that is localized to one part of the object - the "image" that would occur if the object is the face of a human exhaling warm breath. By using the combined signal from two or several areas, it is possible to distinguish between translational movement and variations in shape of the object. Translational movement manifests itself in a time difference between signal signatures representing nearby but different fields of view. On the other hand, signal signatures originating from variations in shape will occur simultaneously in two nearby fields of view.

According to one embodiment the line-of-sight detector 108 is a passive IR sensor operating in the wavelength range 5-8 µm, the output signal of which corresponds to the average temperature of an object within its field of view, the following signal characteristics are valid. The difference between the plateau level 21 and the background level 20 corresponds to the difference between the person's facial skin temperature, and the average temperature of the background when no person's face is within the field of view of the line-of-sight detector 108.

According to one embodiment the line-of-sight detector 108 includes a light emitting diode illuminating a predefined field of view, and a photodiode arranged to detect the reflected light from basically the same field of view, the background and upper levels 20, 21, respectively, corresponds to the average reflectance of the objects within that field of view. Disturbance from extraneous light sources can be minimized by synchronous operation between the light emitting diode and the photodiode and by selecting the operational wavelength range to 0.8-1.0 µm, slightly above the visible range.

A common property in embodiments of the line-of-sight detector 108 is that electromagnetic radiation is employed in either a passive or an active mode of operation. The line-of-sight detector 108 of the embodiments comprise an aperture which directly or indirectly is determining the actual field of view. To avoid undesired effects of wave diffraction this aperture is preferably significantly larger than the operating wavelength range.

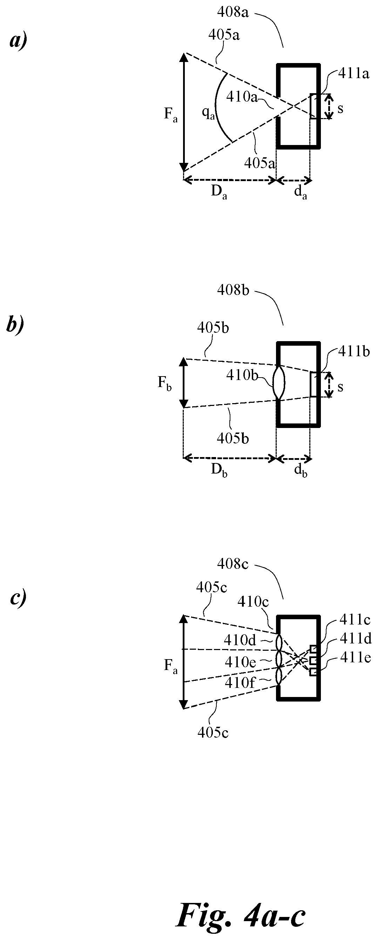

Figure 4a-c illustrates the line-of-sight detector 108 in three alternative embodiments including the optical arrangement 110 defining the field of view 109, and the sensor 111 in the form of a multilayer microstructure 111.

It is understood that several passive and active sensor principles can be employed for the sensor 111. Infrared thermometry is one example of a passive sensor, in which the narrow skin temperature range of a human face provides a useful contrast to the background. The use of near infrared (NIR) reflectance measurements is an example of an active sensor. The field of view in this case is defined by a well-defined, diverging beam of NIR radiation. The presence of a diffusely reflecting object within the field of view provides a reflectance signal that may be detected by a photodiode in the immediate vicinity of the NIR radiator.

The line-of-sight detector arrangement 408a in Figure 4 a) is based on a line-of-sight principle including an opening 410a having a width w defining the field of view 405a.

The predefined distance Dₐ between the face of the person to be examined having a width Fₐ and the opening 410a defines the field of view 405a by the angle qₐ, wherein tan qₐ/2=Fₐ/2Dₐ. Furthermore, the width w of the opening 410a, the size s of the microstructure 411a, and the distance dₐ between the outer surface of the breath analysis device 100 and the surface of the microstructure 411a are given by the equation (1). $w = {d}_{a} \times \frac{{F}_{a}}{{D}_{a}} - s$ (1)

By using equation (1), w can be determined when Fₐ, Dₐ, dₐ, and s are known. Using numerical values of Fₐ =150 mm, Dₐ =100 mm, s=0.3 mm, and dₐ=0.5 mm, an opening width w =0.45 mm is appropriate. The arrangement illustrated in Figure 3 a) is thus appropriate for relatively small distances Dₐ. A human adult face typically has an oval shape having approximate widths of 150 and 200 mm in the horizontal and vertical directions, respectively. The shape of the opening 410a can preferentially be correspondingly oval or elliptical.

One embodiment of a line-of-sight detector arrangement 408b is schematically illustrated in Figure 3 b) and comprises an optical arrangement 410b, which is appropriate at longer distances between the face of the person and the outer surface of the housing 101 of the breath analysis device 100. The optical arrangement 410b comprises a refractive lens 410b provided to focus the heat radiation from the person's face F_{b} to the sensor 411b, preferably a microstructure. The distances D_{b}, and d_{b} are defined as shown in Figure 3b). The focal length f of the lens can be calculated from equation (2) $\frac{1}{f} = \frac{1}{{D}_{b}} + \frac{1}{{d}_{b}}$ (2)

The relation between the size s of the microstructure 11b is given by equation (3) $\frac{{F}_{b}}{s} = \frac{{D}_{b} - f}{f}$ (3)

Inserting the following numerical values D_{b}=300 mm, d_{b}=0.6 mm, and s=0.3 mm results in f=0.6 mm, and F_{b}=150 mm. The optical arrangement 110b thus provides an adequate field of view for a distance D_{b}=300 mm between the person's face having a width F_{b}=150 mm and the outer surface of the breath analysis device 101.

The lens 410b material should preferably have a refractive index of 1.5-1.7, and low absorption in the wavelength range 5-8 µm in the case of a passive IR thermometry sensor, and 0.8-1.0 µm in the case of a NIR reflectance sensor. Polyethylene and polyester (Mylar^{™}) are examples of polymers having adequate infrared transmission characteristics. An appropriate spherical or parabolic lens shape can be obtained by injection molding or die casting of the polymer material.

One embodiment of a line-of-sight detector arrangement 408c is schematically illustrated in Figure 4c and comprises an optical arrangement 410c with multiple lenses 410d, 410e, 410f, each exhibiting different fields of view and focal lengths allowing the radiation heat detection to be divided into separate areas, where multiple sensors 411c, 411d, 411e, preferably microstructures, are located within one or several image planes to allow for different apertures and angular views. As appreciated by the skilled person the depicted arrangement with three lenses and three sensors is a non-limiting example. A larger number of lenses/sensors may be provided and for example provided in a 2D-array. By adding information about the angular heat distribution, more precise analysis of the combined signals from the sensors 411c, 411d, 411e can be obtained.

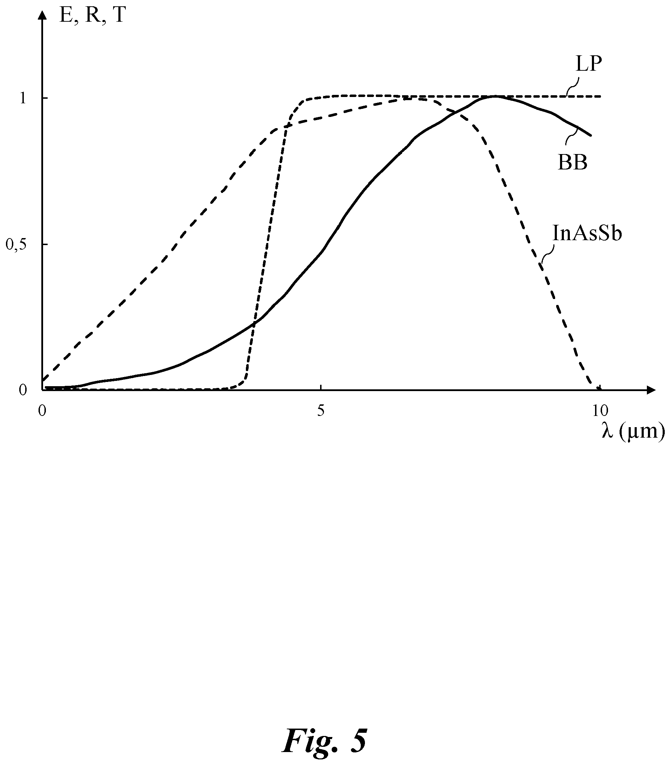

Figure 5 shows a graph of the infrared spectral distribution of heat radiation from a black body, BB, at a temperature of 35°C within the wavelength range of l= 2 ... 10 µm, according to Planck's equation of blackbody radiation. Human skin exhibits an emissivity factor approximately 0.95 regardless of skin color, and therefore the calculated heat emission from a black body is an accurate approximation of heat radiation from human skin. When temperature is increasing from 35°C, the graph denoted BB will shift towards shorter wavelengths and will also increase in magnitude, as evident from Planck's equation. Measurement of the radiated power in one or several wavelength intervals will thus be an indirect measurement of the surface temperature.

Also shown in the graph of Figure 5 is the spectral response of a photovoltaic multilayer microstructure based on the III-V semiconductor indium arsenide antimonide, InAsSb which advantageously may be used as the sensor 111. The multilayered microstructure includes at least one layer exhibiting depletion of charge carriers, electrons or holes. A depletion layer can be generated by a pn-junction between p- or n-dopant materials, or by a Schottky barrier. When photons with appropriate energy is absorbed within this layer, electric charge and a voltage is generated. The detailed response characteristics of the curve denoted InAsSb in Figure 5 is determined by the energy band gap of the semiconductor material which can be modified by substitution between the elements of arsenic, As, and antimony, Sb. By such variations of composition, the downslope occurring at long wavelengths may be shifted to shorter wavelengths by as much as 1.5 µm.

By combining several microstructures having different spectral distributions compared to the curve InAsSb, the precision of skin temperature measurements can be greatly improved. Photovoltaic microstructures with adequate properties are commercially available from Asahi Kasei Microdevices, Tokyo, Japan, product line AK9752, AK9754, AK9756.

The photovoltaic microstructure may include a sensor element responsive to heat conduction in addition to the photovoltaic element responsive to heat radiation. Advantageously, it may also include a Peltier element allowing the microstructure to be heated or cooled to a preset and controlled temperature.

The spectral transmission curve of a long pass filter, LP, is also included in the graph of Figure 5. A long pass filter LP may be inserted in the optical path of the sensor 111 to minimize its cross sensitivity to interference from sources emitting at shorter wavelengths. Long pass or band pass transmission characteristics can be realized using a range of inorganic and polymer materials (S. Musikant Optical Materials, 1985, pp. 176-199).

The spectral distributions in Figure 5 are normalized to the maxima of emission (E), response (R), and transmission (T) of the black body radiator, the photovoltaic microstructure, and the long pass filter, respectively.

A sensor 111 in form of a photovoltaic microstructure has significant advantages compared with, for example, thermopiles operating by the thermoelectric principle, in providing true DC response. Thermoelectric devices require a temperature difference between two locations or more, setting a low frequency limit on the operability. Limitations due to heat conduction and miniaturized design thus make thermoelectric devices less attractive compared to photovoltaic structures. A further advantage of photovoltaic microstructures is superior resolution resulting in higher signal to noise ratio.

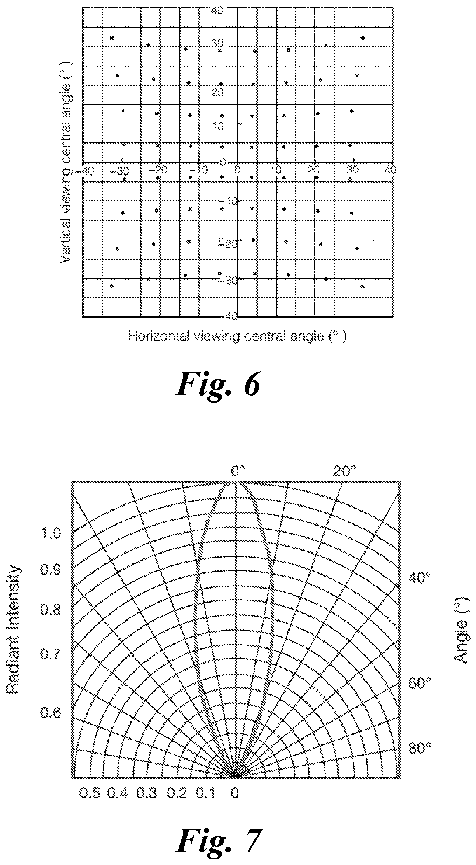

Figure 6 and Figure 7 show representations of the field of view obtained with two types of devices as examples of the physical implementation of the present invention.

The graph of Figure 6 shows the horizontal and vertical angular distributions of the field of view obtained with a commercially available 8x8 matrix of IR photodetectors (Panasonic AMG88). The line-of-sight detector according to this implementation allows the field of view to be divided into a low resolution image. The total field of view of ±30 degrees in each one of the horizontal and vertical directions may be divide into 8 x 8 = 64 individual elements allowing the line-of-sight signal to be composed of several combinations of signals from each one of the 64 elements.

The graph of Figure 7 represents one example of the field of view provided by a line-of-sight detector including a commercially available light emitting diode and a photodiode monitoring the reflectance of objects in the near vicinity (VCNL 3040, Vishay Semiconductors, Inc.). As shown in the graph, the field of view is represented by an annular reflectance distribution with 50% decline at ±15 degrees.

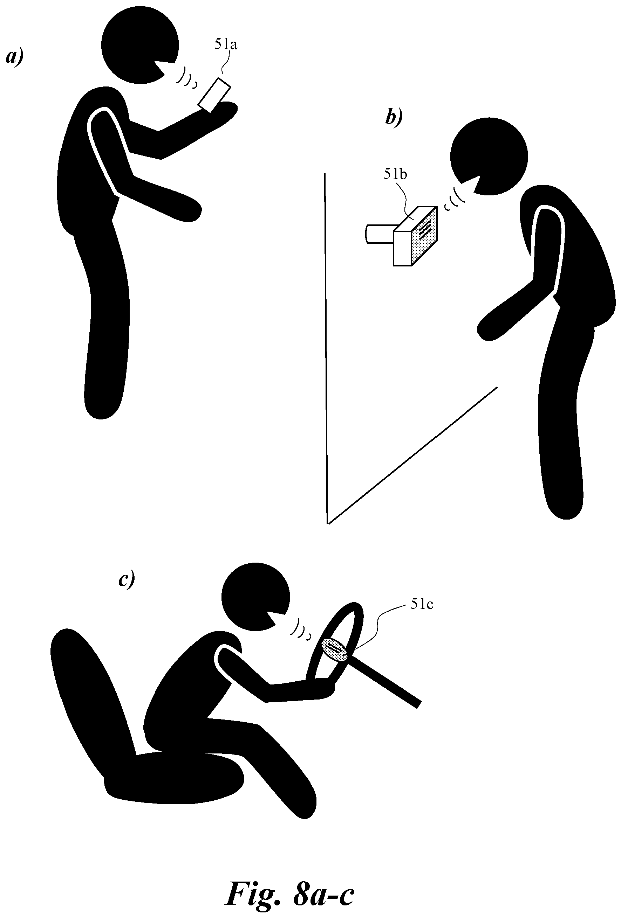

Figure 8 illustrates typical operation of the breath analysis device according to the invention in which the device may be embedded in a handheld device 81a as illustrated in Figure 8a, a wall-mounted free-standing instrument 51b as illustrated in Figure 8b or a unit 51c integrated in, for example, the steering wheel (illustrated) or dash board of a vehicle, as illustrated in Figure 8c.

As illustrated in Figure 8a, in the case of a handheld device 51a, the user is instructed via the communicating unit 113 to grasp and hold the device 51a in his/her hand, bring it within a predefined distance, and then exhale towards it.

As illustrated in Figure 8b, in the case of a wall-mounted free-standing instrument 51b, the user is instructed via the communicating unit 113 to approach the instrument 51b until a predefined distance is reached, and then exhale towards it.

As illustrated in Figure 8c, in the case of a unit 51c integrated within the steering wheel of a vehicle, or similar, the person to be examined is instructed via the communicating unit 113 to lean over towards the unit 51c, thereby bringing it within a predefined distance, and then exhale towards it.

The change of relative distance between the user and the breath analysis device 100, being a result of either the user bringing the device closer or leaning towards the device will results in line-of-sight detector signals in accordance with the graphs of Figure 2a-c. The details in the line-of-sight detector signal signature will differ depending on the implementation which is meant to be accounted for in the establishment of the reference signal stored reference signal signature utilized in the method discussed with reference to Figure 3. The skilled person would easily come up with a suitable reference signal signature relating to a specific implementation typically and preferably based on a limited set of experiments, wherein users test the equipment.

The embodiments described above are to be understood as illustrative examples of the system and method of the present invention. It will be understood that those skilled in the art that various modifications, combinations and changes may be made to the embodiments. In particular, different part solutions in the different embodiments can be combined in other configurations, where technically possible.

## Claims

1. A method of using a breath analysis device (100) for determining the validity of a measurement of a concentration of an intoxicating substance in the exhaled breath of a user,
the breath analysis device (100) comprising:
- a measuring cell arranged to sample a sensor signal representing the concentration of the intoxicating substance and a sensor signal representing the concentration of a tracer substance;
- a line-of-sight detector with a predetermined field of view and arranged to measure the coverage by an object of the field of view and to output a signal representing the degree of coverage,
the method comprising the steps of:
- (305) monitoring the output signal of the line-of-sight detector (108), and if (310) the output signal deviates from an established background value record (315) the line-of-sight detector output signal as a function of time, the output signal variation being a recorded line-of-sight signal signature;
- (320) monitoring the tracer substance sensor signal and if (325) a peak in the tracer substance is detected, the peak indicating a possible exhalation phase of the respiratory cycle of the user determine (330) a breath concentration value of the intoxicating substance based on the intoxicating substance sensor signal and the tracer substance sensor signal;
- (335) comparing the recorded line-of-sight signal signature originating from the line-of-sight detector (108) with at least one stored reference signal signature;
- (345) comparing the relation between the line-of-sight detector output signal and the tracer substance signal with stored signal relation criteria; and wherein
- (340, 350, 355) the validity of the measurement of the breath concentration value is confirmed if the recorded line of sight signal signature matches the stored reference signal signature and if the recorded line-of-sight output signal and the tracer substance signal fulfils the stored signal relation criteria,
**characterized in that** the stored signal relation criteria comprise a time relation between the line-of-sight detector output signal and the tracer substance signal, and
**in that** the line-of-sight detector (108) comprises a sensor (111) arranged to measure heat radiation utilizing infra-red detection.

2. The method according to claim 1, wherein in the monitoring step (305) a background value of the line-of-sight detector output signal is determined, and in the step (335) of comparing the recorded line of sight signal signature with the stored reference signal signature comprises at least comparing one or a selection of the parameters: signal slope after an initial ascension, duration of a time period of increasing signal, a signal value associated to the upper value (21), duration of the signal at or above a predefined level relating to the upper level (21), and signal slope after a peak or plateau.

3. The method according to claim 1, wherein the time relation criterion requires that an increase in the recorded line-of-sight detector output signal, indicative of an object approaching the breath analysis device (100) occurs before the onset of the peak in the tracer substance signal.

4. The method according to claim 3, wherein the time relation criterion requires that a peak in the recorded line-of-sight detector output signal coincide with the peak in the tracer substance signal within a predetermined time period.

5. The method according to claim 4, wherein the step (345) of comparing the relation between the line-of-sight detector output signal and the tracer substance signal with stored signal relation criteria comprises correcting the time difference of the peak in the line-of-sight detector output signal and the peak in the tracer substance by a predetermined factor relating to an expected time delay relating to the time required for an exhaled breath sample reaching the breath analysis device (100).

6. The method according to claim 1, wherein the time relation between the line-of-sight detector output signal and the tracer substance signal includes an expected time differences between the peak in the line-of-sight detector output signal and the peak in the tracer substance by a predetermined factor relating to an expected time delay relating to the time required for an exhaled breath sample reaching the breath analysis device (100).

7. The method according to any of the preceding claims, wherein the line-of-sight detector (108) is arranged to measure heat radiation.

8. The method according to any of the preceding claims, wherein the breath analysis device (100) further comprises means for measuring ambient temperature, and wherein the output signal from the line-of-sight detector (108) is compensated with the ambient temperature.

9. A breath analysis device (100) arranged to perform a measurement of a concentration of an intoxicating substance in the exhaled breath of a user and to the validate the measurement,
the breath analysis device (100) comprising:
- a measuring cell (102) arranged to sample a sensor signal representing the concentration of the intoxicating substance and a sensor signal representing the concentration of a tracer substance;
- a line-of-sight detector (108) with a predetermined field of view and arranged to measure the coverage by an object of the field of view and to output a signal representing the degree of coverage;
- a control and signal processing unit (114) in connection with the measuring cell (102) and the line-of-sight detector (108), wherein the breath analysis device (100) is arranged to perform the steps of:
- (305) monitoring the output signal of the line-of-sight detector (108), and if (310) the output signal deviates from an established background value record (315) the line-of-sight detector output signal as a function of time, the output signal variation being a recorded line-of-sight signal signature;
- (320) monitoring the tracer substance sensor signal and if (325) a peak in the tracer substance is detected, the peak indicating a possible exhalation phase of the respiratory cycle of the user determine (330) a breath concentration value of the intoxicating substance based on the intoxicating substance sensor signal and the tracer substance sensor signal;
- (335) comparing the recorded line-of-sight signal signature originating from the line-of-sight detector (108) with at least one stored reference signal signature;
- (345) comparing the relation between the line-of-sight detector output signal and the tracer substance signal with stored signal relation criteria; and wherein
- (340, 350, 355) the validity of the measurement of the breath concentration value is confirmed if the recorded line of sight signal signature matches the stored reference signal signature and if the recorded line-of-sight output signal and the tracer substance signal fulfils the stored signal relation criteria,
**characterized in that** the stored signal relation criteria comprise a time relation between the line-of-sight detector output signal and the tracer substance signal, and
**in that** the line-of-sight detector (108) comprises a sensor (111) arranged to measure heat radiation utilizing infra-red detection.

10. The breath analysis device (100) according to claim 9, wherein the line-of-sight detector (108) comprises an aperture determining an effective field of view of the line-of-sight detector (108).

11. The breath analysis device (100) according to claim 9, wherein the sensor (111) of the line-of-sight detector (108) is an active sensor arranged to utilize near infrared reflectance measurements.

12. The breath analysis device (100) according to any of claims 9 to 11, wherein the line-of-sight detector (108) is arranged to have a field of view corresponding to a predefined area at a predefined distance from the breath analysis apparatus, wherein the predefined area is the area of a typical human face of a person using the breath analysis device (100) at the predefined distance, the predefined distance associated with appropriate use of breath analysis device (100) and being between 100-300 mm.

13. The breath analysis device (100) according to any of claims 9 to 12, wherein the breath analysis device (100) further comprises means for measuring ambient temperature, and wherein a signal from the line-of-sight detector (108) is compensated with the ambient temperature.

14. The breath analysis device (100) according to any of claims 9 to 13, wherein the line-of-sight detector (108) comprises a plurality of sensors (411c,d,e) arranged to provide a corresponding plurality of output signals providing a spatial resolution of the field of view.

15. The breath analysis device (100) according to any of claims 9 to 13, wherein the line-of-sight detector (108) comprises a 8x8 matrix of IR photodetectors.

16. The breath analysis device (100) according to any of claims 9 to 13, wherein in the monitoring step (305) a background value of the line-of-sight detector output signal is determined, and in the step (335) of comparing the recorded line of sight signal signature with the stored reference signal signature comprises at least comparing one or a selection of the parameters: signal slope after an initial ascension, duration of a time period of increasing signal, a signal value associated to the upper value (21), duration of the signal at or above a predefined level relating to the upper level (21), and signal slope after a peak or plateau.

17. The breath analysis device (100) according to claim 9, wherein the time relation criterion requires that an increase in the recorded line-of-sight detector output signal, indicative of an object approaching the breath analysis device (100) occurs before the onset of the peak in the tracer substance signal.

18. The breath analysis device (100) according to claim 17, wherein the time relation criterion requires that a peak in the recorded line-of-sight detector output signal coincide with the peak in the tracer substance signal within a predetermined time period.

19. The breath analysis device (100) according to claim 18, wherein the step (345) of comparing the relation between the line-of-sight detector output signal and the tracer substance signal with stored signal relation criteria comprises correcting the time difference of the peak in the line-of-sight detector output signal and the peak in the tracer substance by a predetermined factor relating to an expected time delay relating to the time required for an exhaled breath sample reaching the breath analysis device (100).

20. The breath analysis device (100) according to claim 9, wherein the time relation between the line-of-sight detector output signal and the tracer substance signal includes an expected time differences between the peak in the line-of-sight detector output signal and the peak in the tracer substance by a predetermined factor relating to an expected time delay relating to the time required for an exhaled breath sample reaching the breath analysis device (100).

## Patentansprüche

1. Verfahren zum Verwenden einer Atemluftanalysevorrichtung (100), um die Gültigkeit einer Messung einer Konzentration eines Rauschmittels in der Ausatemluft eines Benutzers zu bestimmen, wobei die Atemluftanalysevorrichtung (100) Folgendes umfasst:
- eine Messzelle, die dazu ausgelegt ist, ein Sensorsignal, das die Konzentration des Rauschmittels darstellt, und ein Sensorsignal, das die Konzentration einer Markersubstanz darstellt, abzutasten;
- einen Sichtliniendetektor mit einem vorbestimmten Sichtfeld, der dazu ausgelegt ist, die Abdeckung des Sichtfelds durch ein Objekt zu messen und ein Signal auszugeben, das den Abdeckungsgrad darstellt, wobei das Verfahren die folgenden Schritte umfasst:
- (305) Überwachen des Ausgangssignals des Sichtliniendetektors (108) und, falls (310) das Ausgangssignal von einem festgelegten Hintergrundwert abweicht, Aufzeichnen (315) des Sichtliniendetektor-Ausgangssignals in Abhängigkeit von der Zeit, wobei die Ausgangssignalschwankung eine aufgezeichnete Sichtliniensignalsignatur ist;
- (320) Überwachen des Markersubstanz-Sensorsignals und, falls (325) ein Höchstwert bei der Markersubstanz erkannt wird, wobei der Höchstwert eine mögliche Ausatmungsphase des Atmungszyklus des Benutzers angibt, Bestimmen (330) eines Atemluftkonzentrationswerts des Rauschmittels basierend auf dem Rauschmittel-Sensorsignal und dem Markersubstanz-Sensorsignal;
- (335) Vergleichen der aufgezeichneten Sichtliniensignalsignatur, die von dem Sichtliniendetektor (108) stammt, mit mindestens einer gespeicherten Referenzsignalsignatur;
- (345) Vergleichen der Beziehung zwischen dem Sichtliniendetektor-Ausgangssignal und Markersubstanzsignal mit gespeicherten Signalbeziehungskriterien; und wobei
- (340, 350, 355) die Gültigkeit der Messung des Atemluftkonzentrationswerts bestätigt wird, falls die aufgezeichnete Sichtliniensignalsignatur mit der gespeicherten Referenzsignalsignatur übereinstimmt und falls das aufgezeichnete Sichtlinien-Ausgangssignal und das Markersubstanzsignal die gespeicherten Signalbeziehungskriterien erfüllen, **dadurch gekennzeichnet, dass** die gespeicherten Signalbeziehungskriterien eine zeitliche Beziehung zwischen dem Sichtliniendetektor-Ausgangssignal und dem Markersubstanzsignal umfassen, und dadurch, dass der Sichtliniendetektor (108) einen Sensor (111) umfasst, der dazu ausgelegt ist, Wärmestrahlung unter Verwendung von Infrarotdetektion zu messen.

2. Verfahren nach Anspruch 1, wobei in dem Überwachungsschritt (305) ein Hintergrundwert des Sichtliniendetektor-Ausgangssignals bestimmt wird und in dem Schritt (335) von Vergleichen der aufgezeichneten Sichtliniensignalsignatur mit der gespeicherten Referenzsignalsignatur mindestens Vergleichen eines oder einer Auswahl der folgenden Parameter umfasst ist: Signalsteigung nach einem anfänglichen Anstieg, Dauer eines Zeitraums mit zunehmendem Signal, ein mit dem oberen Wert (21) verknüpfter Signalwert, Dauer des Signals auf oder über einem vordefinierten Pegel, der sich auf den oberen Pegel (21) bezieht, und Signalsteigung nach einem Höchstwert oder Plateau.

3. Verfahren nach Anspruch 1, wobei das Zeitbeziehungskriterium erfordert, dass eine Zunahme bei dem aufgezeichneten Sichtliniendetektor-Ausgangssignal, die auf ein sich der Atemluftanalysevorrichtung (100) näherndes Objekt hinweist, vor dem Einsetzen des Höchstwerts bei dem Markersubstanzsignal auftritt.

4. Verfahren nach Anspruch 3, wobei das Zeitbeziehungskriterium erfordert, dass ein Höchstwert bei dem aufgezeichneten Sichtliniendetektor-Ausgangssignal mit dem Höchstwert bei dem Markersubstanzsignal innerhalb eines vorbestimmten Zeitraums zusammenfällt.

5. Verfahren nach Anspruch 4, wobei der Schritt (345) von Vergleichen der Beziehung zwischen dem Sichtliniendetektor-Ausgangssignal und dem Markersubstanzsignal mit gespeicherten Signalbeziehungskriterien Korrigieren der Zeitdifferenz zwischen dem Höchstwert bei dem Sichtliniendetektor-Ausgangssignal und dem Höchstwert bei der Markersubstanz um einen vorbestimmten Faktor umfasst, der sich auf eine erwartete Zeitverzögerung bezieht, die mit der Zeit zusammenhängt, die eine ausgeatmete Atemluftprobe benötigt, um die Atemluftanalysevorrichtung (100) zu erreichen.

6. Verfahren nach Anspruch 1, wobei die zeitliche Beziehung zwischen dem Sichtliniendetektor-Ausgangssignal und dem Markersubstanzsignal eine erwartete Zeitdifferenzen zwischen dem Höchstwert bei dem Sichtliniendetektor-Ausgangssignal und dem Höchstwert bei der Markersubstanz um einen vorbestimmten Faktor beinhaltet, der sich auf eine erwartete Zeitverzögerung bezieht, die mit der Zeit zusammenhängt, die eine ausgeatmete Atemluftprobe benötigt, um die Atemluftanalysevorrichtung (100) zu erreichen.

7. Verfahren nach einem der vorhergehenden Ansprüche, wobei der Sichtliniendetektor (108) dazu ausgelegt ist, Wärmestrahlung zu messen.

8. Verfahren nach einem der vorhergehenden Ansprüche, wobei die Atemluftanalysevorrichtung (100) ferner Mittel zum Messen von Umgebungstemperatur umfasst und wobei das Ausgangssignal von dem Sichtliniendetektor (108) um die Umgebungstemperatur kompensiert wird.

9. Atemluftanalysevorrichtung (100), die dazu ausgelegt ist, eine Messung einer Konzentration eines Rauschmittels in der Ausatemluft eines Benutzers durchzuführen und die Messung zu der validieren, wobei die Atemluftanalysevorrichtung (100) Folgendes umfasst:
- eine Messzelle (102), die dazu ausgelegt ist, ein Sensorsignal, das die Konzentration des Rauschmittels darstellt, und ein Sensorsignal, das die Konzentration einer Markersubstanz darstellt, abzutasten;
- einen Sichtliniendetektor (108) mit einem vorbestimmten Sichtfeld, der dazu ausgelegt ist, die Abdeckung des Sichtfelds durch ein Objekt zu messen und ein Signal auszugeben, das den Abdeckungsgrad darstellt;
- eine Steuer- und Signalverarbeitungseinheit (114), die mit der Messzelle (102) und dem Sichtliniendetektor (108) verbunden ist, wobei die Atemluftanalysevorrichtung (100) dazu ausgelegt ist, die folgenden Schritte durchzuführen:
- (305) Überwachen des Ausgangssignals des Sichtliniendetektors (108) und, falls (310) das Ausgangssignal von einem festgelegten Hintergrundwert abweicht, Aufzeichnen (315) des Sichtliniendetektor-Ausgangssignals in Abhängigkeit von der Zeit, wobei die Ausgangssignalschwankung eine aufgezeichnete Sichtliniensignalsignatur ist;
- (320) Überwachen des Markersubstanz-Sensorsignals und, falls (325) ein Höchstwert bei der Markersubstanz erkannt wird, wobei der Höchstwert eine mögliche Ausatmungsphase des Atmungszyklus des Benutzers angibt, Bestimmen (330) eines Atemluftkonzentrationswerts des Rauschmittels basierend auf dem Rauschmittel-Sensorsignal und dem Markersubstanz-Sensorsignal;
- (335) Vergleichen der aufgezeichneten Sichtliniensignalsignatur, die von dem Sichtliniendetektor (108) stammt, mit mindestens einer gespeicherten Referenzsignalsignatur;
- (345) Vergleichen der Beziehung zwischen dem Sichtliniendetektor-Ausgangssignal und Markersubstanzsignal mit gespeicherten Signalbeziehungskriterien; und wobei
- (340, 350, 355) die Gültigkeit der Messung des Atemluftkonzentrationswerts bestätigt wird, falls die aufgezeichnete Sichtliniensignalsignatur mit der gespeicherten Referenzsignalsignatur übereinstimmt und falls das aufgezeichnete Sichtlinien-Ausgangssignal und das Markersubstanzsignal die gespeicherten Signalbeziehungskriterien erfüllen,
**dadurch gekennzeichnet, dass** die gespeicherten Signalbeziehungskriterien eine zeitliche Beziehung zwischen dem Sichtliniendetektor-Ausgangssignal und dem Markersubstanzsignal umfassen, und
dadurch, dass der Sichtliniendetektor (108) einen Sensor (111) umfasst, der dazu ausgelegt ist, Wärmestrahlung unter Verwendung von Infrarotdetektion zu messen.

10. Atemluftanalysevorrichtung (100) nach Anspruch 9, wobei der Sichtliniendetektor (108) eine Blende umfasst, die ein effektives Sichtfeld des Sichtliniendetektors (108) bestimmt.

11. Atemluftanalysevorrichtung (100) nach Anspruch 9, wobei der Sensor (111) des Sichtliniendetektors (108) ein aktiver Sensor ist, der dazu ausgelegt ist, Nahinfrarot-Reflexionsmessungen zu nutzen.

12. Atemluftanalysevorrichtung (100) nach einem der Ansprüche 9 bis 11, wobei der Sichtliniendetektor (108) dazu ausgelegt ist, ein Sichtfeld aufzuweisen, das einem vordefinierten Bereich in einem vordefinierten Abstand von der Atemluftanalyseeinrichtung entspricht, wobei der vordefinierte Bereich der Bereich eines typischen menschlichen Gesichts einer Person ist, die die Atemluftanalysevorrichtung (100) in dem vordefinierten Abstand benutzt, wobei der vordefinierte Abstand mit einer bestimmungsgemäßen Verwendung der Atemluftanalysevorrichtung (100) verknüpft ist und zwischen 100-300 mm liegt.

13. Atemluftanalysevorrichtung (100) nach einem der Ansprüche 9 bis 12, wobei die Atemluftanalysevorrichtung (100) ferner Mittel zum Messen von Umgebungstemperatur umfasst und wobei ein Signal von dem Sichtliniendetektor (108) um die Umgebungstemperatur kompensiert wird.

14. Atemluftanalysevorrichtung (100) nach einem der Ansprüche 9 bis 13, wobei der Sichtliniendetektor (108) eine Vielzahl von Sensoren (411c,d,e) umfasst, die dazu ausgelegt sind, eine entsprechende Vielzahl von Ausgangssignalen bereitzustellen, die eine räumliche Auflösung des Sichtfelds bereitstellen.

15. Atemluftanalysevorrichtung (100) nach einem der Ansprüche 9 bis 13, wobei der Sichtliniendetektor (108) eine 8x8-Matrix aus IR-Fotodetektoren umfasst.

16. Atemluftanalysevorrichtung (100) nach einem der Ansprüche 9 bis 13, wobei in dem Überwachungsschritt (305) ein Hintergrundwert des Sichtliniendetektor-Ausgangssignals bestimmt wird und in dem Schritt (335) von Vergleichen der aufgezeichneten Sichtliniensignalsignatur mit der gespeicherten Referenzsignalsignatur mindestens Vergleichen eines oder einer Auswahl der folgenden Parameter umfasst ist: Signalsteigung nach einem anfänglichen Anstieg, Dauer eines Zeitraums mit zunehmendem Signal, ein mit dem oberen Wert (21) verknüpfter Signalwert, Dauer des Signals auf oder über einem vordefinierten Pegel, der sich auf den oberen Pegel (21) bezieht, und Signalsteigung nach einem Höchstwert oder Plateau.

17. Atemluftanalysevorrichtung (100) nach Anspruch 9, wobei das Zeitbeziehungskriterium erfordert, dass eine Zunahme bei dem aufgezeichneten Sichtliniendetektor-Ausgangssignal, die auf ein sich der Atemluftanalysevorrichtung (100) näherndes Objekt hinweist, vor dem Einsetzen des Höchstwerts bei dem Markersubstanzsignal auftritt.

18. Atemluftanalysevorrichtung (100) nach Anspruch 17, wobei das Zeitbeziehungskriterium erfordert, dass ein Höchstwert bei dem aufgezeichneten Sichtliniendetektor-Ausgangssignal mit dem Höchstwert bei dem Markersubstanzsignal innerhalb eines vorbestimmten Zeitraums zusammenfällt.

19. Atemluftanalysevorrichtung (100) nach Anspruch 18, wobei der Schritt (345) von Vergleichen der Beziehung zwischen dem Sichtliniendetektor-Ausgangssignal und dem Markersubstanzsignal mit gespeicherten Signalbeziehungskriterien Korrigieren der Zeitdifferenz zwischen dem Höchstwert bei dem Sichtliniendetektor-Ausgangssignal und dem Höchstwert bei der Markersubstanz um einen vorbestimmten Faktor umfasst, der sich auf eine erwartete Zeitverzögerung bezieht, die mit der Zeit zusammenhängt, die eine ausgeatmete Atemluftprobe benötigt, um die Atemluftanalysevorrichtung (100) zu erreichen.

20. Atemluftanalysevorrichtung (100) nach Anspruch 9, wobei die zeitliche Beziehung zwischen dem Sichtliniendetektor-Ausgangssignal und dem Markersubstanzsignal eine erwartete Zeitdifferenzen zwischen dem Höchstwert bei dem Sichtliniendetektor-Ausgangssignal und dem Höchstwert bei der Markersubstanz um einen vorbestimmten Faktor beinhaltet, der sich auf eine erwartete Zeitverzögerung bezieht, die mit der Zeit zusammenhängt, die eine ausgeatmete Atemluftprobe benötigt, um die Atemluftanalysevorrichtung (100) zu erreichen.

## Revendications

1. Procédé d'utilisation d'un dispositif d'analyse d'haleine (100) pour la détermination de la validité d'une mesure d'une concentration d'une substance intoxicante dans l'haleine exhalée d'un utilisateur, le dispositif d'analyse d'haleine (100) comprenant :
- une cellule de mesure agencée pour l'échantillonnage d'un signal de capteur représentant la concentration de la substance intoxicante et d'un signal de capteur représentant la concentration d'une substance traceuse ;
- un détecteur de ligne de visée avec un champ de vision prédéterminé et agencé pour la mesure de la couverture par un objet du champ de vision et pour la délivrance en sortie d'un signal représentant le degré de couverture, le procédé comprenant les étapes de :
- (305) la surveillance du signal de sortie du détecteur de ligne de visée (108), et si (310) le signal de sortie s'écarte d'une valeur de fond établie, l'enregistrement (315) du signal de sortie du détecteur de ligne de visée en fonction du temps, la variation de signal de sortie étant une signature de signal de ligne de visée enregistrée ;
- (320) la surveillance du signal de capteur de substance traceuse et si (325) un pic dans la substance traceuse est détecté, le pic indiquant une possible phase d'exhalation du cycle respiratoire de l'utilisateur, la détermination (330) d'une valeur de concentration d'haleine de la substance intoxicante sur la base du signal de capteur de substance intoxicante et du signal de capteur de substance traceuse ;
- (335) la comparaison de la signature de signal de ligne de visée enregistrée provenant du détecteur de ligne de visée (108) avec au moins une signature de signal de référence stockée ;
- (345) la comparaison de la relation entre le signal de sortie du détecteur de ligne de visée et le signal de substance traceuse avec des critères de relation de signal stockés ; et dans lequel
- (340, 350, 355) la validité de la mesure de la valeur de concentration d'haleine est confirmée si la signature de signal de ligne de visée enregistrée correspond à la signature de signal de référence stockée et si le signal de sortie de ligne de visée enregistré et le signal de substance traceuse satisfont aux critères de relation de signal stockés, **caractérisé en ce que** les critères de relation de signal stockés comprennent une relation temporelle entre le signal de sortie du détecteur de ligne de visée et le signal de substance traceuse, et **en ce que** le détecteur de ligne de visée (108) comprend un capteur (111) agencé pour la mesure d'un rayonnement thermique en utilisant une détection infrarouge.

2. Procédé selon la revendication 1, dans lequel, dans l'étape de surveillance (305), une valeur de fond du signal de sortie du détecteur de ligne de visée est déterminée, et dans l'étape (335) de comparaison de la signature de signal de ligne de visée enregistrée avec la signature de signal de référence stockée, il est compris au moins la comparaison d'un paramètre ou d'une sélection des paramètres : la pente de signal après une ascension initiale, la durée d'une période de temps d'augmentation de signal, une valeur de signal associée à la valeur supérieure (21), la durée du signal au niveau ou au-dessus d'un niveau prédéfini se rapportant au niveau supérieur (21), et la pente de signal après un pic ou un plateau.

3. Procédé selon la revendication 1, dans lequel le critère de relation temporelle exige qu'une augmentation du signal de sortie du détecteur de ligne de visée enregistré, indicative d'un objet s'approchant du dispositif d'analyse d'haleine (100), se produise avant le début du pic dans le signal de substance traceuse.

4. Procédé selon la revendication 3, dans lequel le critère de relation temporelle exige qu'un pic dans le signal de sortie du détecteur de ligne de visée enregistré coïncide avec le pic dans le signal de substance traceuse dans une période de temps prédéterminée.

5. Procédé selon la revendication 4, dans lequel l'étape (345) de comparaison de la relation entre le signal de sortie du détecteur de ligne de visée et le signal de substance traceuse avec des critères de relation de signal stockés comprend la correction de la différence de temps du pic dans le signal de sortie du détecteur de ligne de visée et du pic dans la substance traceuse par un facteur prédéterminé se rapportant à un retard de temps attendu se rapportant au temps requis pour qu'un échantillon d'haleine exhalée atteigne le dispositif d'analyse d'haleine (100).

6. Procédé selon la revendication 1, dans lequel la relation temporelle entre le signal de sortie du détecteur de ligne de visée et le signal de substance traceuse inclut des différences de temps attendues entre le pic dans le signal de sortie du détecteur de ligne de visée et le pic dans la substance traceuse par un facteur prédéterminé se rapportant à un retard de temps attendu se rapportant au temps requis pour qu'un échantillon d'haleine exhalée atteigne le dispositif d'analyse d'haleine (100).

7. Procédé selon l'une quelconque des revendications précédentes, dans lequel le détecteur de ligne de visée (108) est agencé pour la mesure du rayonnement thermique.

8. Procédé selon l'une quelconque des revendications précédentes, dans lequel le dispositif d'analyse d'haleine (100) comprend en outre des moyens pour la mesure de la température ambiante, et dans lequel le signal de sortie provenant du détecteur de ligne de visée (108) est compensé avec la température ambiante.

9. Dispositif d'analyse d'haleine (100) agencé pour la réalisation d'une mesure d'une concentration d'une substance intoxicante dans l'haleine exhalée d'un utilisateur et pour la validation de la mesure, le dispositif d'analyse d'haleine (100) comprenant :
- une cellule de mesure (102) agencée pour l'échantillonnage d'un signal de capteur représentant la concentration de la substance intoxicante et d'un signal de capteur représentant la concentration d'une substance traceuse ;
- un détecteur de ligne de visée (108) avec un champ de vision prédéterminé et agencé pour la mesure de la couverture par un objet du champ de vision et pour la délivrance en sortie d'un signal représentant le degré de couverture ;
- une unité de commande et de traitement de signal (114) en connexion avec la cellule de mesure (102) et le détecteur de ligne de visée (108), dans lequel le dispositif d'analyse d'haleine (100) est agencé pour la réalisation des étapes de :
- (305) la surveillance du signal de sortie du détecteur de ligne de visée (108), et si (310) le signal de sortie s'écarte d'une valeur de fond établie, l'enregistrement (315) du signal de sortie du détecteur de ligne de visée en fonction du temps, la variation de signal de sortie étant une signature de signal de ligne de visée enregistrée ;
- (320) la surveillance du signal de capteur de substance traceuse et si (325) un pic dans la substance traceuse est détecté, le pic indiquant une possible phase d'exhalation du cycle respiratoire de l'utilisateur, la détermination (330) d'une valeur de concentration d'haleine de la substance intoxicante sur la base du signal de capteur de substance intoxicante et du signal de capteur de substance traceuse ;
- (335) la comparaison de la signature de signal de ligne de visée enregistrée provenant du détecteur de ligne de visée (108) avec au moins une signature de signal de référence stockée ;
- (345) la comparaison de la relation entre le signal de sortie du détecteur de ligne de visée et le signal de substance traceuse avec des critères de relation de signal stockés ; et dans lequel
- (340, 350, 355) la validité de la mesure de la valeur de concentration d'haleine est confirmée si la signature de signal de ligne de visée enregistrée correspond à la signature de signal de référence stockée et si le signal de sortie de ligne de visée enregistré et le signal de substance traceuse satisfont aux critères de relation de signal stockés,
**caractérisé en ce que** les critères de relation de signal stockés comprennent une relation temporelle entre le signal de sortie du détecteur de ligne de visée et le signal de substance traceuse, et
**en ce que** le détecteur de ligne de visée (108) comprend un capteur (111) agencé pour la mesure d'un rayonnement thermique en utilisant une détection infrarouge.

10. Dispositif d'analyse d'haleine (100) selon la revendication 9, dans lequel le détecteur de ligne de visée (108) comprend une ouverture déterminant un champ de vision effectif du détecteur de ligne de visée (108).

11. Dispositif d'analyse d'haleine (100) selon la revendication 9, dans lequel le capteur (111) du détecteur de ligne de visée (108) est un capteur actif agencé pour l'utilisation de mesures de réflectance dans le proche infrarouge.

12. Dispositif d'analyse d'haleine (100) selon l'une quelconque des revendications 9 à 11, dans lequel le détecteur de ligne de visée (108) est agencé pour avoir un champ de vision correspondant à une zone prédéfinie à une distance prédéfinie de l'appareil d'analyse d'haleine, dans lequel la zone prédéfinie est la zone d'un visage humain typique d'une personne utilisant le dispositif d'analyse d'haleine (100) à la distance prédéfinie, la distance prédéfinie étant associée à une utilisation appropriée du dispositif d'analyse d'haleine (100) et étant comprise entre 100 et 300 mm.

13. Dispositif d'analyse d'haleine (100) selon l'une quelconque des revendications 9 à 12, dans lequel le dispositif d'analyse d'haleine (100) comprend en outre des moyens pour la mesure de la température ambiante, et dans lequel un signal provenant du détecteur de ligne de visée (108) est compensé avec la température ambiante.

14. Dispositif d'analyse d'haleine (100) selon l'une quelconque des revendications 9 à 13, dans lequel le détecteur de ligne de visée (108) comprend une pluralité de capteurs (411c,d,e) agencés pour la fourniture d'une pluralité correspondante de signaux de sortie fournissant une résolution spatiale du champ de vision.

15. Dispositif d'analyse d'haleine (100) selon l'une quelconque des revendications 9 à 13, dans lequel le détecteur de ligne de visée (108) comprend une matrice 8x8 de photodétecteurs IR.

16. Dispositif d'analyse d'haleine (100) selon l'une quelconque des revendications 9 à 13, dans lequel, dans l'étape de surveillance (305), une valeur de fond du signal de sortie du détecteur de ligne de visée est déterminée, et dans l'étape (335) de comparaison de la signature de signal de ligne de visée enregistrée avec la signature de signal de référence stockée, il est compris au moins la comparaison d'un paramètre ou d'une sélection des paramètres : la pente de signal après une ascension initiale, la durée d'une période de temps d'augmentation de signal, une valeur de signal associée à la valeur supérieure (21), la durée du signal au niveau ou au-dessus d'un niveau prédéfini se rapportant au niveau supérieur (21), et la pente de signal après un pic ou un plateau.

17. Dispositif d'analyse d'haleine (100) selon la revendication 9, dans lequel le critère de relation temporelle exige qu'une augmentation du signal de sortie du détecteur de ligne de visée enregistré, indicative d'un objet s'approchant du dispositif d'analyse d'haleine (100), se produise avant le début du pic dans le signal de substance traceuse.

18. Dispositif d'analyse d'haleine (100) selon la revendication 17, dans lequel le critère de relation temporelle exige qu'un pic dans le signal de sortie du détecteur de ligne de visée enregistré coïncide avec le pic dans le signal de substance traceuse dans une période de temps prédéterminée.

19. Dispositif d'analyse d'haleine (100) selon la revendication 18, dans lequel l'étape (345) de comparaison de la relation entre le signal de sortie du détecteur de ligne de visée et le signal de substance traceuse avec des critères de relation de signal stockés comprend la correction de la différence de temps du pic dans le signal de sortie du détecteur de ligne de visée et du pic dans la substance traceuse par un facteur prédéterminé se rapportant à un retard de temps attendu se rapportant au temps requis pour qu'un échantillon d'haleine exhalée atteigne le dispositif d'analyse d'haleine (100).

20. Dispositif d'analyse d'haleine (100) selon la revendication 9, dans lequel la relation temporelle entre le signal de sortie du détecteur de ligne de visée et le signal de substance traceuse inclut une différence de temps attendue entre le pic dans le signal de sortie du détecteur de ligne de visée et le pic dans la substance traceuse par un facteur prédéterminé se rapportant à un retard de temps attendu se rapportant au temps requis pour qu'un échantillon d'haleine exhalée atteigne le dispositif d'analyse d'haleine (100).
